# FASCICULE DE BREVET EUROPEEN

(11) **EP 0 954 341 B1**
(45) Date de publication et mention de la délivrance du brevet: **28.02.2007**
(21) Numéro de dépôt: 97951333.0
(22) Date de dépôt: 15.12.1997
(51) Int. Cl.: A61L 27/54, A61F 2/16

(54) **LENTILLE INTRAOCULAIRE CONTENANT UN MEDICAMENT RELARGABLE**
INTRAOKULARE LINSE MIT ABZUGEBENDEM MEDIKAMENT
INTRAOCULAR LENS CONTAINING RELEASABLE MEDICATION

(30) Priorité: 13.12.1996 FR 9615356
(43) Date de publication de la demande: 10.11.1999
(73) Titulaire: Ioltechnologie-Production, 17180 Perigny (FR)
(72) Inventeur: AIACHE, Jean-Marc, F-63000 Clermont-Ferrand (FR); SERPIN, Gilbert, F-63000 Clermont-Ferrand (FR); EL MESKI, Said, F-21 000 Dijon (FR); TOURRETTE, Philippe, F-17220 Clavette (FR)
(74) Mandataire: Santarelli
(86) Numéro de dépôt international: PCT/FR1997/002297
(87) Numéro de publication internationale: WO 1998/025652

(56) Documents cités:
- EP-A- 0 335 785
- EP-A- 0 443 809
- EP-A- 0 563 984
- US-A- 4 240 163
- US-A- 5 554 187
- CHAPMAN J.M. ET AL.: "DRUG INTERACTION WITH INTRAOCULAR LENSES OF DIFFERENT MATERIALS" J.CATARACT REFRACTIVE SURGERY, vol. 18, no. 5, 1992, pages 456-459, XP002039994 cité dans la demande
- NISHI O. ET AL.: "EFFECT OF INDOMETHACIN COATED POSTERIOR CHAMBER INTRAOCULAR LENSES ON POSTOPERATIVE INFLAMMATION AND POSTERIOR CAPSULE OPACIFICATION" JOURNAL OF CATARACT & REFLECTIVE SURGERY, vol. 21, no. 5, 1995, pages 574-578, XP002039995 cité dans la demande
- HEYRMAN T.P. ET AL.: "DRUG UPTAKE AND RELEASE BY A HYDROGEL INTRAOCULAR LENS AND THE HUMAN CRYSTALLINE LENS" J. CATARACT REFRACTIVE SURGERY, vol. 15, no. 2, 1989, pages 169-175, XP002039996 cité dans la demande
- EL MESKI ET AL.: "Use of Methyl Polymethacrylate (PMMA) as a Drug Support" 1ST WORLD MEETING APGI APV BUDAPEST, 9 mai 1995 (1995-05-09), pages 323-324, BUDAPEST

## Description

L'invention se rapporte à une lentille destinée à être implantée dans l'oeil d'un sujet, pour être substituée à un cristallin défectueux au cours d'une opération chirurgicale, lentille réalisée en un polymère hydrophile.

De telles lentilles, dites lentilles intraoculaires, en abrégé "LIO" ou cristallins artificiels sont largement utilisées, notamment pour remédier à l'opacification du cristallin dite aphaque.

Le succès de telles interventions nécessite, compte tenu de l'acte chirurgical lui-même, ainsi que de la nature des produits utilisés, l'administration concomitante de divers médicaments pour limiter et atténuer les risques d'inflammation.

Le traitement correspondant comprend l'instillation (2 à 4 fois par jour, parfois plus) dans l'oeil, de médicaments adaptés, notamment anti-inflammatoires, pendant une période de temps prolongée, allant jusqu'à un mois.

Etant donné que les membranes externes de l'oeil, cornée et sclérotique, représentent des barrières au passage des produits médicamenteux, on est amené à administrer des médicaments à posologie bien plus élevée, les excédents étant soit perdus, soit, ce qui est plus grave, drainés par le liquide lacrymal pour passer ensuite dans la circulation systémique, et donc augmenter les effets indésirables et toxiques.

L'invention a pour objectif de profiter de ce que, lors de l'implantation, la sclérotique est incisée pour livrer passage à la lentille, pour optimiser la thérapeutique par diminution des prises médicamenteuses et des risques d'effets secondaires, en libérant les principes actifs sur les sites mêmes d'action.

Il a été proposé [Journal of Cataract & Refractive Surgery, vol. 21, N° 5, 1995, (Nishi et al) : "Effect of indomethacin coated posterior chamber intraocular lenses on postoperative inflammation and posterior capsule opacification", EP-A-0.563.984 (Unitika Ltd)] de former un dépôt superficiel d'un polymère contenant des anti-inflammatoires ou analogues sur une LIO, pour faire agir ce médicament à l'emplacement précis où son action est requise. Mais le médicament est en quantité faible (l'épaisseur du recouvrement est nécessairement faible), et est relargué dans l'humeur aqueuse pratiquement extemporanément, et son action ne se prolonge guère plus que celle d'un médicament injecté directement dans cette humeur aqueuse lors de l'intervention. Les auteurs visent à pallier les réactions immédiates de l'oeil à l'implantation. Ces traitements de surface ne dispensent pas d'un traitement post-opératoire classique, avec ses inconvénients évoqués ci-dessus.

Pour atteindre cet objectif, l'invention propose une lentille destinée à être implantée dans l'oeil d'un sujet, notamment pour être substituée à un cristallin défectueux au cours d'une opération chirurgicale, lentille réalisée en polymère pour lentille souple ayant un taux d'hydrophilie donné, caractérisée en ce que le polymère contient, dispersé dans sa masse, , en quantité efficace, un produit médicamenteux, à effets appropriés, notamment inhibition au moins partielle des réactions post-opératoires de l'oeil, l'association du polymère et du produit médicamenteux dispersé étant apte à relarguer progressivement le produit dans l'humeur aqueuse et tissus intraoculaires et en ce que la lentille est en condition stérilisée.

Comme les polymères utilisés pour réaliser les lentilles sont hydrophiles, le produit médicamenteux va se relarguer progressivement dans l'humeur aqueuse, et sera ainsi à portée immédiate de ses sites d'action.

Par polymère hydrophile, on entend ici un polymère susceptible de contenir un certain taux d'eau interne, pour permettre le relargage des produits actifs dans l'humeur aqueuse. Le taux en poids d'eau interne, ou taux d'hydrophilie, doit être au moins de 0,20 % et de préférence, d'au moins 0,25 %.

Le document EP-A-0 594 948 décrit un implant, capable de libérer un médicament approprié, dans l'orbite ou le globe oculaire. Cet implant, qui a une forme allongée en bague ouverte, est constitué d'un polymère biodégradable dans la masse duquel un médicament a été incorporé. La libération de ce médicament résulte d'une biodégradation. Ce mode de fonctionnement est manifestement incompatible avec son incorporation dans une lentille intraoculaire, qui est implantée pour durer et ne doit donc pas subir une dégradation. On observera que ce document EP-A-0 594 948 prétend que l'implant qu'il décrit pourrait être utilisé comme élément de construction d'une lentille intraoculaire. Il apparaît qu'un tel élément ne peut constituer un élément fonctionnel de la lentille (optique et haptiques ou anses), en raison de son caractère biodégradable et donc essentiellement temporaire.

Le document EP-A-0.335.785 décrit des polymères prévus pour constituer des lentilles de contact qui comportent des constituants classiques d'un hydrogel et un monomère porteur de produit actif copolymérisable avec lesdits constituants, ledit monomère comportant un radical aryle dont une fonction phénol est estérifiée par un composé à fonction acide du type de l'indométhacine constituant ledit produit actif. Ce monomère peut être en particulier l'indométhacine de méthacrylamido-4-phénol.

La publication "Drug Uptake and Release by an Hydrogel Intraocular Lens and Human Crystalline Lens (Heyrman T.P. et al), parue dans Journal of Cataract & Refractive Surgery, vol. 15, N° 2, 1989, étudie le comportement de LIO en polyméthylméthacrylate, comparé avec des yeux d'êtres vivants (lapins et hommes), vis-à-vis de médicaments, notamment anti-inflammatoires, utilisés pour le traitement opératoire et post-opératoire de la chirurgie de la cataracte. Les essais, in vitro et (sur les lapins seuls) in vivo montrent que l'hydrogel absorbe les médicaments, en quantité et à une vitesse très voisines de celles qu'absorbent les cristallins des yeux, et que les relargages dans l'humeur aqueuse (et vitrée) s'opèrent dans des conditions semblables. Les auteurs concluent que les LIO étudiées ne peuvent opérer comme des réserves significatives de médicaments dans l'oeil.

La publication "Drug Interaction with Intraocular Lenses of Different Materials" (J.M. Chapman et al), parue dans Journal of Cataract & Refractive Surgery, vol. 18, N° 5 (1992), complète des études antérieures sur les interactions de médicaments avec des LIO en PMMA et polyhydroxyéthylméthacrylate (dont la publication précédente), en considérant d'autres matériaux et d'autres médicaments. Cette publication conclut que les LIO ne peuvent apporter des quantités suffisantes de médicaments dans l'oeil pour modifier de façon sensible la cinétique des médicaments en applications topiques, sous-conjonctivales ou intraveineuses, et remplacer les traitements classiques.

La communication "Use of Methyl Polymethacrylate (PMMA) as a Drug Support" El Meski, Beyssac and Aiache; Proc. 1st World Meeting APGI/APV, Budapest 9/11 May 1995, pages 323-324, enseigne un procédé d'incorporation d'un médicament dans du PPMA, comportant un séchage du polymère, une absorption d'une solution de médicament dans un véhicule eau/éthanol à 57% en masse d'éthanol, et un séchage final (5 jours à 110°C) pour évacuer le véhicule.

Le passage des enseignements de cette communication au procédé défini ci-dessus a nécessité, d'une part, qu'on ait envisagé, de façon originale, d'utiliser le matériau constitutif d'une lentille intraoculaire dure comme réservoir d'un produit médicamenteux relargable dans l'humeur aqueuse, et les tissus oculaires environnants et, d'autre part, de façon imprévisible, que le polymère traité suivant les enseignements de la communication précitée permettrait de disposer d'une lentille optiquement et chirurgicalement apte à se substituer à un cristallin, et cela de façon durable.

La communication constatait que la diffusion du véhicule dans le polymère induisait des transformations profondes de structure de l'implant, passage de l'état vitreux dur à un état élastique analogue à du caoutchouc lors de l'imprégnation, puis retour à l'état vitreux par évaporation du véhicule.

En outre, la transposition du traitement d'une matière pour lentille dure à celui d'une matière pour lentille souple, qui est implantée à l'état gonflé, présentait des inconnues sérieuses, en raison du comportement différent des polymères vis-à-vis des solvants susceptibles de constituer des véhicules, et des différences de vitesses de diffusion et de relargage propres à ces types de polymères.

Les travaux des demandeurs ont fait apparaître que, pour une mise en oeuvre efficace de l'objet de l'invention, il convenait d'adapter certaines opérations du procédé à la nature du polymère utilisé pour la lentille.

Ainsi, dans le cas d'un polymère pour lentille dure, il convenait d'effectuer l'imprégnation notamment à température élevée sur une ébauche qui est ensuite conformée ou usinée en lentille intraoculaire, pour avoir une partie optique sans imperfections de surface, et des parties haptiques non fragilisées.

Dans le cas d'un polymère pour lentille souple, il convient de stériliser la lentille conformée dans une solution isotonique à concentration choisie en produit médicamenteux.

Cette stérilisation peut permettre, à elle seule, l'imprégnation. En variante l'imprégnation précède la stérilisation. La concentration du produit médicamenteux est choisie en fonction de la charge désirée de la lentille en produit médicamenteux, et des propriétés réciproques du polymère et du produit médicamenteux.

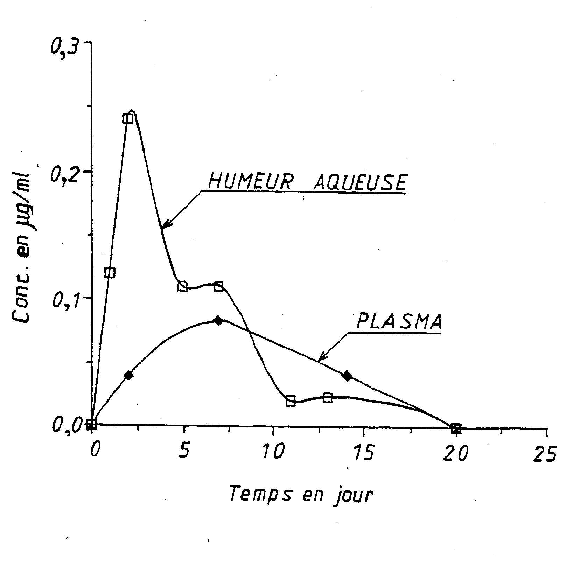

Des caractéristiques secondaires et des avantages de l'invention ressortiront d'ailleurs de la description qui va suivre, assortie d'exemples et en référence à la figure unique annexée.

Cette figure unique représente la variation au cours du temps de la concentration en indométhacine dans l'humeur aqueuse et dans le plasma d'un lapin auquel a été implantée une lentille intraoculaire selon l'invention.

Les premiers essais de mise au point de l'invention ont porté sur des lentilles intraoculaires en polymère pour lentilles dures, à savoir PMMA. L'imprégnation de lentilles intraoculaires, suivant les principes exposés dans la communication précitée de El Meski, Beyssac et Aiache, dans leur conformation définitive, a fait apparaître une fragilisation des anses haptiques, et des défauts des surfaces dioptriques, de sorte que les lentilles présentaient des qualités insuffisantes.

L'invention est définie par les revendications indépendantes portant sur des lentilles intraoculaires souples et dures et sur des procédés de préparer celles-ci.

### EXEMPLE 1. Préparation de lentilles dures en PMMA

On a donc opéré sur des ébauches en forme de disques ou palets, qui ont subi le traitement suivant :
- Séchage des ébauches en étuve à 110°C pendant 24 heures, pour évacuer l'eau interne ;
- Immersion, à température élevée, pendant 24 heures, dans une solution d'un véhicule eau/éthanol à 57 % en poids d'éthanol, saturée en diclofenac, contenue dans un récipient opaque, et maintenue à 50°C ;
- Rinçage dans un mélange eau/alcool à 50 % ;
- Séchage à 110°C pendant 5 jours ;
- Découpage par usinage en lentilles intraoculaires conformées ;
- Polissage des surfaces dioptriques ;
- Nettoyage et stérilisation.

### EXEMPLE 2 Préparation de lentilles imprégnées d'indométhacine

Des ébauches en PMMA ont subi le même traitement qu'à l'exemple 1, à la différence que le véhicule eau/ éthanol à 57 % d'éthanol était saturé en indométhacine. Pour le reste, les opérations ont été identiques.

Divers essais, effectués par relargage in vitro sur des ébauches traitées, mais non conformées en LIO, ont fait apparaître que les doses de produit médicamenteux contenues dans les ébauches étaient de l'ordre de grandeur nécessaire, et que la vitesse de relargage dans une solution isotonique (comparable en comportement à l'humeur aqueuse (vitrée) devait assurer des périodes d'activité de durée convenable.

### EXEMPLE 3. Cinétique de libération chez le lapin

Trois lentilles intraoculaires en PMMA imprégnées d'indométhacine suivant l'exemple 2 ont été implantées chacune en substitution du cristallin dans un oeil d'un lapin, l'oeil controlatéral devant servir de témoin.

On a prélevé de l'humeur aqueuse des yeux traités à JO (avant implantation) J7, J14, J21 et J28, soit cinq prélèvements par lapin.

L'indométhacine à été dosée par chromatographie liquide à haute performance.

Cliniquement, les examens ont montré que, dès le dixième jour de l'implantation, les réactions inflammatoires ont disparu, et que l'oeil traité est identique à l'oeil témoin.

Pratiquement, les dosages d'indométhacine sur les prélèvements d'humeur aqueuse n'ont pas donné de résultats quantifiables ; seuls les prélèvements 3 et 4 d'un lapin (deuxième et troisième semaines) ont fait apparaître des traces appréciables, de l'ordre de 0,25 µg/ml.

On peut donc conclure que les résultats cliniques sont satisfaisants alors que les doses relarguées ou absorbées après relargage sont du même ordre que la limite quantifiable. Cet exemple vient souligner l'intérêt de localiser la distribution du produit médicamenteux à proximité immédiate des sites d'action, afin de limiter les doses de produit instillé dans l'oeil et d'éviter que ce produit puisse avoir une action indésirable dans les parties non intéressées de l'organisme.

Les exemples suivants se rapportent à des lentilles intraoculaires souples, notamment en poly-HEMA (poly-hydroxyéthylméthacrylate).

### EXEMPLE 4. Imprégnation de poly-HEMA (Cet exemple ne comprend pas d'étape de stérilisation telle que définie dans les revendications.):

Une lentille intraoculaire en poly-HEMA (contenant 38 % en poids d'eau), prête pour l'implantation, est extraite de son étui de livraison, pesée et séchée en étuve à 50°C pendant 75 minutes. Sa masse n'est plus que de 62 % de sa masse d'origine, montrant que l'eau contenue est sensiblement totalement évacuée.

La lentille est alors immergée dans un véhicule eau/éthanol à 57 % en masse d'éthanol, saturé en indométhacine, et maintenu à 50°C. Après 1 5 minutes d'immersion, la masse de la lentille est de 11 5 % de la masse d'origine (185 % de la masse à l'état sec) ; après 35 minutes, de 130 % de la masse d'origine (200 % de la masse à l'état sec) ; après 45 minutes, de 150 % de la masse d'origine (240 % de la masse à l'état sec).

Des essais similaires, sur des lentilles séchées pendant des temps plus courts, ont montré que le pourcentage de gonflement dans le véhicule eau/éthanol saturé en produit médicamenteux, est fonction de la durée et du taux de séchage. Il est ainsi possible de régler la quantité de produit médicamenteux fixé dans la lentille.

### EXEMPLE 5. Préparation d'une implantation

Une lentille intraoculaire imprégnée comme à l'exemple 4 est lavée dans une solution tampon à 0,9 % de NaCl (solution isotonique), à pH 7,0, saturée en indométhacine, puis placée dans un étui individuel de transport contenant de la solution tampon précédente, saturée en indométhacine. L'étui contenant la lentille est stérilisé à l'autoclave à 120°C pendant 30 minutes.

Le contrôle de lentilles en fin de préparation montre qu'elles n'ont pas subi d'altérations ; toutefois elles présentent une teinte jaune qui est une preuve de la présence d'indométhacine.

### EXEMPLE 6. Cinétique de libération chez le lapin

Une lentille préparée comme à l'exemple 5 est implantée dans la chambre antérieure de l'oeil droit d'un lapin. On prélève, à intervalles arbitraires, des échantillons d'humeur aqueuse (environ 150 *µ*l) et de plasma (2 ml environ). Sur ces échantillons, l'indométhacine est dosée par chromatographie liquide à haute performance (HPLC). Les résultats de ces dosages sont indiqués sur la courbe de la figure unique annexée.

On constate que l'indométhacine est décelable jusqu'au vingtième jour. La concentration en indométhacine dans l'humeur aqueuse présente un pic principal (0,25 µg/ml environ) vers le troisième jour, et un pic secondaire peu accusé (0,1 1 µg/ml environ), vers le septième jour. Dans le plasma, la concentration croît progressivement jusqu'au septième jour (0,9 µg/ml environ), pour décroître ensuite régulièrement jusqu'au vingtième jour.

Les constatations cliniques ont montré que les lentilles étaient bien tolérées, et que les réactions inflammatoires disparaissaient au moins aussi rapidement que dans le cas de l'exemple 3.

Des essais analogues ont été entrepris avec des lentilles intraoculaires souples en polymères autres, tels que "polymère acrylique". Par "polymère acrylique", on entend ici le polymère couramment désigné ainsi dans le domaine des lentilles intraoculaires.

On peut même envisager des polymères en silicones. Par "silicones", on entend des polymères utilisés dans le domaine intraoculaire et présentant un taux minimum d'hydrophilie par exemple au moins 0,20 %. A cette fin, le polymère à base de silicone subit une transformation physico-chimique afin de raccourcir les chaînes, de sorte que le polymère sous forme de liquide permettra l'imprégnation du produit médicamenteux. Il va de soi que le polymère ainsi imprégné devra ensuite être conformé en lentille intraoculaire.

On a constaté que l'imprégnation, après un séchage approprié pour évacuer l'eau interne, pouvait être réalisée pendant la stérilisation, les lentilles étant enfermées dans des étuis de transport contenant, en guise de véhicule, une solution isotonique tamponnée à pH 7,2, et à concentration choisie en produit médicamenteux.

La stérilisation est alors effectuée en autoclave à 120°C, pendant 25 minutes.

### Exemple 7 (Cet exemple ne comprend pas d'étape de stérilisation telle que définie dans les revendications):

On a recherché les conditions dans lesquelles introduire des quantités efficaces de médicament dans des LIO souples en poly-HEMA.

Dans un premier temps, des LIO souples ont été imprégnées par immersion dans une solution saturée en diclofénac (1,8 mg/ml, préparée en dissolvant du diclofénac sodique dans une solution de NaCl à 0,9%), à température ambiante pendant 72 heures, soigneusement rincées puis mises à relarguer dans 1 ml d'une solution de NaCl 0,9%. Après des durées de 6, 24 et 96 heures, le diclofénac est dosé dans la solution de relargage par HPLC, puis la LIO est rincée, et pour les durées intermédiaires, remise à relarguer dans une solution neuve.

| Temps | Quantités fixées en µg/ml | Quantités cumulées en µg/ml |
|---|---|---|
| 6 heures | 127 | 127 |
| 24 heures | 95 | 222 |
| 96 heures | 111 | 333 |

### Exemple 8 :

On a repris l'essai de l'exemple 7 en laissant les LIO dans la solution saturée 7 jours au lieu de 3. Les contrôles ont été effectués à 6 et 24 heures de relargage.

| Temps | Quantités fixées en µg/ml | Quantités cumulées en µg/ml |
|---|---|---|
| 6 heures | 122 | 122 |
| 24 heures | 109 | 231 |

On constate que l'allongement de la durée d'imprégnation dans la solution saturée de 3 à 7 jours a été pratiquement sans influence sur les quantités relarguées, ce qui signifie qu'à 72 heures à la température ambiante la LIO est pratiquement saturée.

Les essais suivants ont eu pour but de déterminer de façon plus précise la cinétique de libération du diclofénac (essais *in vitro).*

Comme précédemment, les mesures de quantités relarguées ont été effectuées en prélevant le milieu de relargage (solution neutre de NaCl 0,9%) à intervalles pour dosage, et en le renouvelant pour un nouvel intervalle. Les prélèvements ont eu lieu à 6 heures, 24 heures, puis toutes les 24 heures pendant 14 jours au total. Pour être plus proche des conditions d'utilisation projetée, le relargage a été effectué à 35°C.

L'imprégnation des LIO a été réalisée par deux méthodes différentes. Pour un premier essai, les LIO ont été introduites dans la solution saturée en diclofénac, puis l'ensemble a été stérilisé à 122°C pendant 30 minutes. Un second essai a été conduit sans stérilisation, les LIO restant dans la solution de diclofénac pendant 24 heures.

Les quantités relarguées ont été sensiblement les mêmes à plus ou moins 10% près, et sont données dans le tableau suivant, en heure (H), jour (J) et en µg/ml.

| T | 6H | 24H | 2J | 3J | 4J | 5J | 6J | 7J | 8H | 9J | 10J | 11H | 12J | 13J | 14J. |
|---|---|---|---|---|---|---|---|---|---|---|---|---|---|---|---|
| Q | 95 | 80 | 40 | 30 | 20 | 18 | 12 | 10 | 10 | 11 | 12 | 14 | 16 | 18 | 20 |

On constate ainsi que la quantité de diclofénac à saturation est pratiquement indépendante de la température d'imprégnation, la saturation à température ambiante étant atteinte en moins de 3 jours médicamenteuse quelques minutes, voire quelques heures avant l'implantation, ceci étant vrai pour les implants à haut pouvoir d'hydratation en poly-HEMA ou polymère acrylique. De même, la stérilisation est effectuée avant l'imprégnation, ce qui empêchera toute dénaturation du produit médicamenteux et notamment ceux susceptibles de se dénaturer à température élevée.

Enfin, parmi les solvants susceptibles d'être utilisés comme véhicule d'imprégnation, on peut citer, outre ceux décrits dans les exemples et les essais ci-dessus, dans le groupe des solvants organiques, l'alcool isopropylique et l'acétonitrile ou dans le groupe des solvants aqueux, des solutions salines complexes telles que des solutions d'irrigation intraoculaire, et notamment le BSS.

## Revendications

1. Lentille destinée à être implantée dans l'oeil d'un sujet, notamment pour être substituée à un cristallin défectueux au cours d'une opération chirurgicale, lentille réalisée en polymère pour lentille souple ayant un taux d'hydrophilie donné, **caractérisée en ce que** le polymère contient, dispersé dans sa masse, en quantité efficace, un produit médicamenteux, à effets appropriés, notamment inhibition au moins partielle des réactions post-opératoires de l'oeil, l'association du polymère et du produit médicamenteux dispersé étant apte à relarguer progressivement le produit dans l'humeur aqueuse et tissus intraoculaires et **en ce que** la lentille est en condition stérilisée.

2. Lentille intraoculaire selon la revendication 1, **caractérisée en ce que** le produit médicamenteux, en quantité efficace, a des effets d'inhibition au moins partielle des réactions post-opératoires de l'oeil.

3. Lentille intraoculaire selon la revendication 1 ou 2, **caractérisée en ce que** le produit médicamenteux est un anti-inflammatoire.

4. Lentille intraoculaire selon la revendication 3, **caractérisée en ce que** le produit médicamenteux est choisi parmi l'indométhacine, le diclofenac et la dexaméthasone.

5. Lentille intraoculaire selon l'une des revendications 1, 2 ou 3, où le polymère est un poly-HEMA, polymère acrylique ou polymères en silicones.

6. Lentille intraoculaire selon l'une quelconque des revendications 1 à 5 **caractérisée en ce que** le polymère présente un taux d'hydrophilie d'au moins 0,20 % en poids.

7. Lentille intraoculaire selon l'une quelconque des revendications 1 à 5, **caractérisée en ce que** le polymère présente un taux d'hydrophilie d'au moins 0,25 % en poids.

8. Lentille intraoculaire selon l'une quelconque des revendications 1, 5 à 7, **caractérisée en ce que** le produit médicamenteux est un antibiotique, antimitotique ou autre anti-infectieux,

9. Procédé de préparation d'une lentille intraoculaire à produit médicamenteux relargable après implantation dans l'oeil d'un sujet, **caractérisé en ce que** l'on prépare une ébauche d'un polymère en matière de lentille dite dure et ayant un taux d'hydrophilie donné, qu'on évacue la majeure partie de l'eau absorbée du polymère, et on immerge l'ébauche du polymère au sein d'une solution dosée du produit médicamenteux dans un véhicule apte à imprégner le polymère hydrophile, jusqu'à absorption substantielle de la solution dans ce polymère, **en ce que** après l'immersion de l'ébauche on conforme le polymère en lentille intraoculaire propre à être implantée dans un oeil du sujet, en sorte que le produit médicamenteux se relargue progressivement dans l'humeur aqueuse et tissus intraoculaires, et que l'on stérilise la lentille.

10. Procédé de préparation d'une lentille intraoculaire selon la revendication 9, **caractérisé en ce que** la stérilisation de la lentille est effectuée après l'imprégnation.

11. Procédé de préparation d'une lentille intraoculaire selon la revendication 10, **caractérisé en ce que** l'implant est rincé avec une solution dudit produit médicamenteux dans ledit véhicule.

12. Procédé selon l'une quelconque des revendications 9 à 11, **caractérisé en ce que** l'immersion de l'ébauche dans ladite solution est exécutée alors à une température appropriée en fonction des caractéristiques physico-chimiques du produit médicamenteux.

13. Procédé selon l'une quelconque des revendications 9 à 12, **caractérisé en ce que** le véhicule est constitué d'un mélange d'eau et un solvant organique, notamment alcoolique ou une solution saline.

14. Procédé suivant l'une quelconque des revendications 9 à 13, **caractérisé en ce que** le véhicule est constitué d'un mélange d'eau et d'éthanol, la proportion massique de l'éthanol dans le véhicule étant de 57 %.

15. Procédé selon la revendication 9 à 14, **caractérisé en ce que** après la conformation de l'ébauche en lentille intraoculaire, elle est débarrassée des imperfections de surface, et ensuite nettoyée et stérilisée.

16. Lentille intraoculaire réalisée selon l'une quelconque des revendications 9 à 15.

17. Procédé de préparation d'une lentille intraoculaire en polymère hydrophile pour lentille souple à produit médicamenteux relargable après implantation dans l'oeil d'un sujet, selon la revendication 1 à 8, dans lequel on immerge la lentille au sein d'une solution dosée d'un produit médicamenteux dans un véhicule apte à imprégner le polymère hydrophile jusqu'à l'absorption substantielle de la solution dans le polymère de la lentille et on stérilise la lentille.

18. Procédé de préparation d'une lentille intraoculaire selon la revendication 17, **caractérisé en ce que** la stérilisation est effectuée lors de l'immersion de la lentille dans la solution.

19. Procédé de préparation d'une lentille intraoculaire selon la revendication 17, **caractérisé en ce que** l'imprégnation est effectuée après stérilisation, le polymère étant à haut pouvoir d'hydratation tel que poly-HEMA ou polymère acrylique.

20. Procédé de préparation d'une lentille intraoculaire selon la revendication 17, **caractérisé en ce que** l'imprégnation est effectuée avant stérilisation.

## Claims

1. Lens made from a soft lens polymer having a specified hydrophilic rate intended for implantation in the eye of a subject, and notably for substitution for a defective crystalline lens during a surgical operation, **characterised in that** the polymer contains, dispersed in its mass, a medicated product, in an effective amount, with appropriate effects, in particular at least partial inhibition of postoperative reactions of the eye, the association of the polymer and the dispersed medicated product being adapted to release the product progressively into the aqueous humour and intraocular tissues and **in that** the lens is in a sterilised condition.

2. Intraocular lens according to claim 1, **characterised in that** the medicated product in an effective amount has effects of at least partial inhibition of postoperative reactions of the eye.

3. Intraocular lens according to claim 1 or claim 2, **characterised in that** the medicated product is an anti-inflammatory.

4. Intraocular lens according to claim 3, **characterised in that** the medicated product is chosen from indomethacin, diclofenac and dexamethasone.

5. Intraocular lens according to claim 1 or claim 2 or claim 3 wherein the polymer is a poly-HEMA, acrylic polymer or silicone polymers.

6. Intraocular lens according to any one of claims 1 to 5 **characterised in that** the polymer has a hydrophilic rate of at least 0.20% by weight.

7. Intraocular lens according to any one of claims 1 to 5, **characterised in that** the polymer has a hydrophilic rate of at least 0.25% by weight.

8. Intraocular lens according to any one of claims 1 and 5 to 7, **characterised in that** the medicated product is an antibiotic, antimitotic or other anti-infection agent.

9. Method for preparing an intraocular lens containing a medicated product releasable after implantation in the eye of a subject, **characterised in that** a blank is prepared of a hard lens material polymer having a given hydrophilic rate, most of the absorbed water is eliminated from the polymer, and the polymer blank is immersed in a solution dosed with the medicated product in a vehicle adapted to impregnate the hydrophilic polymer until the solution is substantially absorbed into the polymer, **in that** after immersion of the blank the polymer is conformed into an intraocular lens adapted to be implanted in the eye of the subject, so that the medicated product is released progressively into the aqueous humour and intraocular tissues, and the lens is sterilised.

10. Method for preparing an intraocular lens according to claim 9, **characterised in that** the lens is sterilised after impregnation.

11. Method for preparing an intraocular lens according to claim 10, **characterised in that** the implant is rinsed with a solution of said medicated product in said vehicle.

12. Method according to any one of claims 9 to 11, **characterised in that** the blank is immersed in said solution at an appropriate temperature given the physicochemical characteristics of the medicated product.

13. Method according to any one of claims 9 to 12, **characterised in that** the vehicle is a mixture of water and an organic solvent, in particular an alcohol-based solvent or a saline solution.

14. Method according to any one of claims 9 to 13, **characterised in that** the support is a mixture of water and ethanol and the proportion of ethanol in the support is 57% by weight.

15. Method according to claims 9 to 14, **characterised in that** after the blank has been conformed into an intraocular lens, surface defects are removed and it is then cleaned and sterilised.

16. Intraocular lens made in accordance with any one of claims 9 to 15.

17. Method for preparing an intraocular lens according to any one of claims 1 to 8 made from a hydrophilic soft lens polymer containing a medicated product releasable after implantation in the eye of a subject, wherein the lens is immersed in a solution dosed with a medicated product in a vehicle adapted to impregnate the hydrophilic polymer until the solution is substantially absorbed in the polymer of the lens and the lens is sterilised.

18. Method for preparing an intraocular lens according to claim 17, **characterised in that** sterilisation is effected during immersion of the lens in the solution.

19. Method for preparing an intraocular lens according to claim 17, **characterised in that** impregnation is effected after sterilisation, the polymer, such as poly-HEMA or acrylic polymer, having a high hydrating power.

20. Method for preparing an intraocular lens according to claim 17, **characterised in that** impregnation is effected before sterilisation.

## Patentansprüche

1. Linse für die Implantation in das Äuge einer Person, insbesondere zum Ersetzen einer geschädigten Kristalllinse bei einer chirurgischen Operation, wobei die Linse aus weichem Polymer für weiche Linse mit gegebenem Hydrophiliegrad hergestellt ist, **dadurch gekennzeichnet, dass** das Polymer, dispergiert in seiner Masse, ein Medikamentenprodukt in wirksamer Menge mit geeigneten Wirkungen, insbesondere einer zumindest teilweisen Hemmung der postoperativen Augenreaktionen, enthält, wobei die Verbindung des Polymers und des dispergierten Medikamentenprodukts dazu ausgelegt ist, nach und nach das Produkt im Kammerwasser und intraokularen Geweben zu verteilen, und **dadurch**, dass die Linse im sterilisierten Zustand vorliegt.

2. Intraokularlinse nach Anspruch 1, **dadurch gekennzeichnet, dass** das Medikamentenprodukt in wirksamer Menge zumindest teilweise Wirkungen zur Hemmung der postoperativen Augenreaktionen besitzt.

3. Intraokularlinse nach Anspruch 1 oder 2, **dadurch gekennzeichnet, dass** das Medikamentenprodukt ein entzündungshemmendes Mittel ist.

4. Intraokularlinse nach Anspruch 3, **dadurch gekennzeichnet, dass** das Medikamentenprodukt aus Indomethacin, Diclofenac und Dexamethason ausgewählt ist.

5. Intraokularlinse nach einem der Ansprüche 1, 2 oder 3, wobei das Polymer ein Poly-HEMA, ein Acrylpolymer oder Silikonpolymere ist.

6. Intraokularlinse nach einem der Ansprüche 1 bis 5, **dadurch gekennzeichnet, dass** das Polymer einen Hydrophiliegrad von mindestens 0,20 Gew.-% aufweist.

7. Intraokularlinse nach einem der Ansprüche 1 bis 5, **dadurch gekennzeichnet, dass** das Polymer einen Hydrophiliegrad von mindestens 0,25 Gew.-% aufweist.

8. Intraokularlinse nach einem der Ansprüche 1, 5 bis 7, **dadurch gekennzeichnet, dass** das Medikamentenprodukt ein Antibiotikum, ein Mitosehemmer oder ein anderes antiinfektiöses Mittel ist.

9. Verfahren zur Herstellung einer Intraokularlinse mit einem Medikamentenprodukt, das nach Implantation in das Auge einer Person verteilbar ist, **dadurch gekennzeichnet, dass** ein Vorprodukt aus einem Polymer aus so genanntem hartem Linsenmaterial mit gegebenem Hydrophiliegrad hergestellt, der größte Teil des absorbierten Wassers des Polymers ausgetrieben und die Polymervorform in eine Lösung, die mit dem Medikamentenprodukt in einem Vehikel, das an das Imprägnieren des hydrophilen Polymers angepasst ist, versetzt ist, bis zu einer erheblichen Absorption der Lösung in das Polymer eingetaucht wird, und **dadurch**, dass nach dem Eintauchen der Vorform das Polymer zu einer Intraokularlinse geformt wird, die dazu geeignet ist, in ein Auge der Person implantiert zu werden, derart, dass sich das Medikamentenprodukt nach und nach im Kammerwasser und in den intraokularen Geweben verteilt, und **dadurch**, dass die Linse sterilisiert wird.

10. Verfahren zur Herstellung einer Intraokularlinse nach Anspruch 9, **dadurch gekennzeichnet, dass** die Sterilisation der Linse nach dem Imprägnieren erfolgt.

11. Verfahren zur Herstellung einer Intraokularlinse nach Anspruch 10, **dadurch gekennzeichnet, dass** das Implantat mit einer Lösung des Medikamentenprodukts in dem Vehikel gespült wird.

12. Verfahren nach einem der Ansprüche 9 bis 11, **dadurch gekennzeichnet, dass** das Eintauchen der Vorform in die Lösung dann bei einer geeigneten Temperatur in Abhängigkeit von den physikalisch-chemischen Eigenschaften des Medikamentenprodukts durchgeführt wird.

13. Verfahren nach einem der Ansprüche 9 bis 12, **dadurch gekennzeichnet, dass** das Vehikel aus einem Gemisch von Wasser und einem organischen, insbesondere alkoholischen, Lösungsmittel oder einer Kochsalzlösung besteht.

14. Verfahren nach einem der Ansprüche 9 bis 13, **dadurch gekennzeichnet, dass** das Vehikel aus einem Gemisch von Wasser und Ethanol besteht, wobei der Gewichtsanteil von Ethanol in dem Vehikel 57% beträgt.

15. Verfahren nach einem der Ansprüche 9 bis 14, **dadurch gekennzeichnet, dass** nach dem Formen der Vorform zu einer Intraokularlinse diese von Oberflächenmängeln befreit und anschließend gereinigt und sterilisiert wird.

16. Intraokularlinse, die nach einem der Ansprüche 9 bis 15 hergestellt wird.

17. Verfahren zur Herstellung einer Intraokularlinse aus hydrohpile Polymer für weiche Linse mit einem Medikamentenprodukt, das nach der Implantation in das Auge einer Person verteilbar ist, nach Anspruch 1 bis 8, wobei die Linse in eine Lösung, die mit einem Medikamentenprodukt in einem Vehikel, das dazu geeignet ist, das hydrophile Polymer zu imprägnieren, versetzt ist, bis zu einer erheblichen Absorption der Lösung in das Polymer der Linse eingetaucht und die Linse sterilisiert wird.

18. Verfahren zur Herstellung einer Intraokularlinse nach Anspruch 17, **dadurch gekennzeichnet, dass** die Sterilisation beim Eintauchen der Linse in die Lösung durchgeführt wird.

19. Verfahren zur Herstellung einer Intraokularlinse nach Anspruch 17, **dadurch gekennzeichnet, dass** die Imprägnierung nach der Sterilisation durchgeführt wird, wobei das Polymer ein hohes Hydratationsvermögen hat, wie Poly-HEMA oder Acrylpolymer.

20. Verfahren zur Herstellung einer Intraokularlinse nach Anspruch 17, **dadurch gekennzeichnet, dass** die Imprägnierung vor der Sterilisation durchgeführt wird.
